# DEMANDE DE BREVET EUROPEEN

(11) **EP 1 566 163 A1**
(43) Date de publication de la demande: **24.08.2005**
(21) Numéro de dépôt: 05290113.9
(22) Date de dépôt: 19.01.2005
(51) Int. Cl.: A61K 7/06, A61K 7/48

(54) **Procédé de préparation d'une composition de traitement cosmétique à partir de fluide sous pression et d'esters**

(30) Priorité: 29.01.2004 FR 0400848
(71) Demandeur: L'OREAL, 75008 Paris (FR)
(72) Inventeur: Grollier, Jean-François, 75006 Paris (FR); Dubief, Claude, 78150 Le Chesnay (FR); De la Mettrie, Roland, 78110 Le Vesinet (FR)
(74) Mandataire: Casalonga, Axel

(57) **Abrégé**

La présente invention concerne un procédé de préparation d'une composition de traitement cosmétique des matières kératiniques, par percolation de fluide comprenant au moins de la vapeur d'eau, au travers d'au moins un ester sous forme solide ou pâteuse.

L'invention concerne aussi un dispositif de conditionnement d'une composition contenant un tel ester, ainsi que le procédé de traitement cosmétique mettant en oeuvre une telle composition.

## Description

La présente invention a pour objet un procédé de préparation d'une composition cosmétique destinée au traitement des matières kératiniques, en particulier de la peau et des fibres kératiniques humaines telles que les cheveux, ainsi qu'un procédé de traitement cosmétique des matières kératiniques à partir de cette composition.

En cosmétique on cherche toujours à améliorer les propriétés cosmétiques des matières kératiniques, par exemple des cheveux sensibilisés, i.e. qui sont abîmés ou fragilisés sous l'action chimique des agents atmosphériques tels que les radicaux libres, la lumière et la pollution, et/ou des traitements capillaires tels que permanentes, teintures ou décolorations. On peut alors leur appliquer des compositions de traitement cosmétique comprenant des agents de conditionnement tels que des esters, destinés à réparer ou limiter les effets néfastes ou indésirables produits par les différents traitements ou agressions que subissent, de manière plus ou moins répétée, les matières kératiniques. Ces agents de conditionnement peuvent également améliorer les propriétés cosmétiques des cheveux naturels.

Les agents de conditionnement des matières kératiniques, tels que les esters, confèrent notamment, aux cheveux secs ou mouillés, une bonne aptitude au démêlage, un lissage, une douceur et une brillance.

Ces agents de conditionnement confèrent également à la peau des propriétés cosmétiques telles qu'une bonne hydratation et une bonne nutrition.

Cependant, les compositions de traitement cosmétique contenant de tels agents de conditionnement sont généralement des compositions aqueuses dans lesquelles lesdits agents doivent être solubilisés. Le manque de solubilité de ces composés diminue le pouvoir conditionnant de ces compositions. En outre, ce critère de solubilité réduit le nombre d'esters que l'on peut utiliser pour le traitement cosmétique des matières kératiniques. Ceci est particulièrement le cas des composés à point de fusion élevé.

La Demanderesse a découvert de manière surprenante qu'en utilisant un nouveau procédé de préparation d'une composition de traitement cosmétique des matières kératiniques, on pouvait obtenir en un temps très court, inférieur à 2 minutes, des compositions plus ou moins concentrées en agent(s) de conditionnement selon le besoin, notamment sans conservateur, permettant de surmonter les problèmes de solubilité exposés ci-dessus.

Ce procédé est mis en oeuvre simplement et est approprié au besoin du consommateur. On fait passer un fluide, dont la température est de préférence supérieure ou égale à 30 °C, sous pression, pendant un laps de temps très court, inférieur à 1 minute, à travers au moins un agent de conditionnement sous forme solide ou pâteuse, de préférence solide, et encore plus préférentiellement pulvérulente.

Il permet aussi d'utiliser sous forme anhydre des agents de conditionnement instables dans des compositions aqueuses soit parce qu'ils réagissent avec l'eau, soit parce qu'ils réagissent en solution aqueuse avec des composés qui ne réagissent pas avec eux dans une composition anhydre. C'est notamment le cas des esters.

Les compositions préparées selon ce procédé peuvent présenter une stabilité au stockage limitée, ce qui n'est pas ici un inconvénient puisque le procédé conduit à une composition prête à l'emploi, destinée à être utilisée rapidement après sa préparation, par exemple dans les 5 minutes suivant sa préparation, notamment après refroidissement jusqu'à une température acceptable pour les matières kératiniques, de préférence inférieure à 60 °C, mieux inférieure à 50 °C. La composition peut être également utilisée jusqu'à une semaine après sa préparation, selon la vitesse de dégradation de l'agent de conditionnement utilisé.

Etant donné le temps de préparation très court, les compositions de traitement cosmétique peuvent être préparées "à la demande" en mélangeant différents composés actifs en cosmétique selon les propriétés cosmétiques recherchées.

Selon un autre mode de réalisation, les agents conditionnement pouvant être conditionnés dans un dispositif prêt-à-l'emploi, il n'est pas nécessaire de déterminer au préalable les concentrations desdits agents en solution, ce qui limite les erreurs de mesure de l'utilisateur.

En outre, le procédé selon l'invention permet d'éviter l'utilisation de flacons multi-compartiments, ce qui rend le procédé particulièrement économique et plus sûr pour l'utilisateur.

La composition ainsi obtenue peut être utilisée seule ou en mélange avec une autre composition.

Un avantage supplémentaire de ce procédé de préparation est l'obtention de compositions présentant de meilleures propriétés cosmétiques. En particulier, les fibres kératiniques traitées avec une composition obtenue par le procédé selon l'invention présentent des propriétés de conditionnement améliorées, et notamment de démêlage, de lissage, de brillance et de douceur au toucher, et la peau ainsi traitée présente une douceur, un caractère lisse et une hydratation améliorés.

L'invention a donc pour objet un procédé de préparation d'une composition de traitement cosmétique des matières kératiniques comprenant une étape de percolation d'un fluide sous une pression d'au moins 3 bars au travers d'au moins un ester sous forme solide ou pâteuse.

Un autre objet de l'invention est une composition susceptible d'être obtenue par le procédé selon l'invention.

L'invention a également pour objet l'utilisation de la composition obtenue selon le procédé de l'invention, pour le traitement cosmétique des matières kératiniques, et notamment le conditionnement des cheveux et de la peau.

L'invention a encore pour objet un dispositif de conditionnement permettant de mettre en oeuvre le procédé de préparation de la présente invention.

D'autres objets, caractéristiques, aspects et avantages de l'invention apparaîtront encore plus clairement à la lecture de la description et des exemples qui suivent.

Selon l'invention, le procédé de préparation d'une composition de traitement cosmétique des matières kératiniques comprend une étape de percolation de fluide, à une température de préférence supérieure ou égale à 30 °C, mieux encore allant de 30 à 150 °C, et encore plus préférentiellement de 40 à 120 °C, sous une pression d'au moins 3 bars (3.10⁵ Pa) au travers d'au moins un ester sous forme solide ou pâteuse.

La percolation est un mouvement de fluide à travers un milieu poreux permettant le passage du fluide, sous l'action ou l'effet de la pression.

Le fluide utilisé dans l'invention comprend au moins de la vapeur d'eau. Il peut être constitué par de la vapeur d'eau éventuellement accompagnée d'eau liquide, ou par un mélange de vapeur d'eau éventuellement accompagnée d'eau liquide, et d'un ou plusieurs solvants liquides et/ou gazeux cosmétiquement acceptables. De préférence, il est de la vapeur d'eau pouvant être accompagnée d'eau liquide.

A titre de solvant organique, on peut citer, par exemple, les alcools inférieurs en C₁-C₄ tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

L'ester est sous forme solide ou pâteuse, de préférence sous forme solide, et encore plus préférentiellement pulvérulente.

Par «forme pâteuse» au sens de la présente invention, on entend une consistance intermédiaire entre une phase solide et une phase liquide. La viscosité de cette phase pâteuse est de préférence supérieure à 0,1 Pa.s, et encore plus préférentiellement supérieure à 1 Pa.s, ceci à 25°C avec un taux de cisaillement de 10 s⁻¹.

Par "matières kératiniques", on entend la peau, les lèvres, et/ou les phanères tels que les ongles et les fibres kératiniques, par exemple les cils, les sourcils et les cheveux.

Le procédé de la présente invention peut être mis en oeuvre à partir d'un dispositif classique permettant de générer un fluide sous pression, à une température de préférence supérieure ou égale à 30 °C, mieux encore allant de 30 à 150 °C, et encore plus préférentiellement de 40 à 120 °C. Un tel dispositif comprend une chambre résistant à la pression, équipée d'un bloc thermique, ainsi qu'un circuit d'acheminement du fluide vers l'agent de conditionnement utilisé dans l'invention.

Selon un autre mode de réalisation, le dispositif comprend de plus un réservoir de liquide(s) ainsi qu'une pompe permettant l'acheminement du ou des liquides vers la chambre.

Le liquide contenu dans le réservoir est soit de l'eau, soit un mélange d'eau et d'un ou plusieurs solvants cosmétiquement acceptables. De préférence, le liquide est de l'eau.

Un dispositif particulièrement utile pour la mise en oeuvre du procédé de la présente invention est une machine à café du type "expresso". De telles machines sont bien connues de la technique. Par exemple, ces machines sont décrites dans les brevets AT 168405, US 2688911, DE 32433870 et IT 1265636.

Selon un mode de réalisation particulier de l'invention, l'étape de percolation est mise en oeuvre avec un fluide à une température supérieure ou égale à 30 °C, de préférence allant de 30 à 150 °C, et encore plus préférentiellement de 40 à 120 °C, sous une pression allant de 3 à 30 bars, de préférence d'au moins 4 bars (4.10⁵ Pa), mieux encore supérieure ou égale à 10 bars (10⁶ Pa), et tout particulièrement allant de 10 à 30 bars (10⁶ à 3.10⁶ Pa).

Une composition cosmétique comprenant au moins un ester sous forme solide ou pâteuse peut être utilisée directement dans le dispositif générant le fluide sous pression dans un récipient destiné à cet usage. Elle peut aussi être conditionnée dans un dispositif de conditionnement particulier comprenant un logement fermé délimité par au moins une paroi au moins en partie perméable au fluide sous une pression d'au moins 3 bars. De tels dispositifs sont, par exemple, décrits dans les demandes de brevets WO 00/56629, EP512470, US 5897899 ou WO 99/03573. Ces dispositifs de conditionnement sont en général étanches à l'air, l'humidité et/ou la lumière.

Selon un mode de réalisation particulier, le logement est délimité par deux feuilles scellées. Selon un autre mode de réalisation, le logement est délimité par une barquette fermée par un couvercle.

Ces dispositifs peuvent être fabriqués à partir de matériaux tissés ou non tissés, en matière plastique ou végétale, par exemple en cellulose, en métal tel que l'aluminium ou en composite. De tels dispositifs sont par exemple décrits dans les demandes de brevets WO 00/56629, EP 512470, US 5897899 ou WO 99/03573.

Les esters utilisables dans la présente invention peuvent être monomériques ou polymériques. Ils sont de préférence non siliconés et non cationiques.

A titre d'exemples de ces esters, on peut notamment citer :
- les esters de monoacides carboxyliques en C₇-C₃₀, linéaires ou ramifiés, et de monoalcools en C₈-C₃₀, linéaires ou ramifiés, tels que par exemple, le palmitate de cétyle, le myristate de myristyle, l'heptanoate de stéaryle, le béhénate d'isostéaryle, l'iso-octanoate de cétyle, le 2-décylmyristate de 2-décylmyristyle et le bénénate de bénényle ;
- les esters de monoacides carboxyliques insaturés linéaires ou ramifiés en C₇-C₃₀, ou aromatiques en C₆-C₃₀, et de monoalcools en C₈-C₃₀, linéaires ou ramifiés, tels que par exemple, le benzoate de stéaryle, le ricinoléate de cétyle, l'érucate de 2-octyldodécyle et le benzoate de bénényle ;
- les mono- et diesters de monoacides carboxyliques linéaires ou ramifiés en C₁₀-C₃₀ et d'éthylèneglycol tels que, par exemple, le monostéarate d'éthylèneglycol, le distéarate d'éthylèneglycol et le dimyristate d'éthylèneglycol ;
- les mono-, di- ou triesters de monoacides carboxyliques linéaires ou ramifiés, saturés ou insaturés, en C₁₀-C₃₀ et de polyol en C₃, tels que par exemple, les mono-, di- et tristéarates de glycéryle, les mono-, di- et tribéhénates de glycéryle, le monomyristate de propylèneglycol, le palmitostéarate de glycéryle, le mono-oléate de glycéryle, le tristéarobéhénate de glycéryle, le tri-undécanoate de glycéryle, le beurre de karité, la cire du Japon, le beurre d'Illipe et le beurre de cacao ;
- les mono- ou polyesters de monoacides carboxyliques linéaires ou ramifiés, saturés ou insaturés, ou aromatiques en C₇-C₃₀ et de polyols non polyoxyalkylénés en C₄-C₅₀, et en particulier de sucres. A titre d'exemples, on peut citer le benzoate de saccharose, le tétra-isostéarate de pentaérythrityle, le tétrabéhénate de pentaérythrityle, le tétralaurate de pentaérythrityle, le palmitate de sorbitane, l'o-octanoyl-6'-D-maltose, le monostéarate de sorbitane, le laurate de saccharose, le dibenzoate de néopentylglycol, le tétrabenzoate de pentaérythrityle, les esters d'acides gras de coco et de sorbitol, le tétrastéarate de ditriméthylolpropane, l'hexastéarate de dipentaérythrityle, les mono- et distéarates de méthylglycose, le dilaurate de saccharose, le palmitate de dextrine, les mono- et dipalmitostéarates de saccharose, le sesquistéarate de méthylglucose et le monococoate d'hexylglucoside ;
- les mono-, di- ou triesters de di- ou triacides carboxyliques linéaires ou ramifiés, saturés ou insaturés, aromatiques ou non, en C₄-C₃₀, hydroxylés ou non, et de mono- ou polyols linéaires ou ramifiés en C₇-C₅₀. On peut citer à titre d'exemples, le fumarate de dibéhényle, l'ester citrique de monoglycérides de suif, le citrate de dicocoyl-distéaryl-penarylthrityle, le tartrate de dimyristile, les mucates de dihexyle, dioctyle, dilauryle, dicétyle, les citrates de di- et trilauryle ;
- les mono- ou polyesters d'acides et/ou d'alcools polyoxyalkylénés ou polycérolés, la portion non-oxyalkylénée ou non-glycérolée des acides étant en C₁₀-C₃₀, tels que l'huile de jojoba à 80 moles d'oxyde d'éthylène (ou OE) et la cire de jojoba à 120 moles d'OE, le myristate de myristyle à 3 moles d'OE, les mono- et distéarates de triéthylèneglycol, les monostéarates de polyéthylèneglycol à 8, 25, 30, 100 moles d'OE, les distéarates de polyéthylèneglycol à 8, 150 et 250 moles d'OE, le dibéhénate de polyéthylèneglycol à 150 moles d'OE, le diisostéarate de polyéthylèneglycol à 90 moles d'OE, les cires d'abeille oxyéthylénées à 6, 8 ou 12 moles d'OE, le monostéarate de sorbitane oxyalkyléné à 4 moles d'OE, le triisostéarate de sorbitane oxyéthyléné à 200 moles d'OE, l'hexastéarate de sorbitane oxyéthyléné à 6 moles d'OE, le dioléate de méthylglucoside oxyéthyléné à 120 moles d'OE, le beurre de karité oxyéthyléné à 75 moles d'OE, l'huile de ricin oxyéthylénée à 25 moles d'OE, le palmitostéarate polyglycérolé à 6 moles de glycérol ;
- les huiles hydrogénées ou cires hydrogénées telles que par exemple, les huiles hydrogénées de colza, de macadamia, de ricin, de coton, de soja, de jojoba, de palme, de son, de riz, de tournesol, la cire de tournesol hydrogénée ;
- les sels des acides résultant de l'estérification de monoacides carboxyliques linéaires ou ramifiés, saturés ou insaturés, en C₇-C₃₀ et d'hydroyacides. Parmi ceux-ci, on peut citer le lauroyllactylate de sodium, le béhénoyllactylate de sodium ;
- les polymères dont au moins l'un des monomères est un ester. Ces polymères peuvent être des homopolymères ou des copolymères. Ils peuvent être de nature non ionique, anionique, cationique ou amphotère et peuvent, en particulier, être obtenus par polymérisation radicalaire.
   Parmi les homopolymères, on peut citer les poly(méthacrylates de méthyle), les poly(acétates de vinyle), les polycaprolactones, les poly(acrylates de stéaryle), les poly(acrylates d'éthyle).
   Parmi les copolymères, on peut citer les copolymères d'acétate de vinyle et d'éthylène, les copolymères acétate de vinyle/versatate de vinyle/acrylate de butyle, les copolymères acide acrylique/ méthacrylate de stéaryle, les copolymères N-octylacrylamide/méthacrylate de méthyle/méthacrylate d'hydroxypropyle/ acide acrylique/méthacrylate de tertiobutylaminoéthyle, les copolymères acide lactique/vinylpyrrolidone/méthacrylate de lauryle ; et
- les polymères résultant de la polycondensation d'au moins un polyacide et d'au moins un polyol. Parmi ces polymères, on peut citer le poly(téréphtalate de néopentylglycol), les polymères de diglycol/cyclohexanediméthanol/isophtalates/sulfoisophtalates, les polyesters de polyglycérol et d'acide eicosanedioique, les polyesters de triméthylolpropane et d'acide diméthylolpropionique, les polymères de polyéthylèneglycol et d'acide adipique, les polymères d'acide sébacique et de butylèneglycol.

Les esters particulièrement préférés dans la présente invention sont choisis parmi les esters de monoacides carboxyliques en C₇-C₃₀, linéaires ou ramifiés, et de monoalcools en C₈-C₃₀, linéaires ou ramifiés ; les esters de monoacides carboxyliques insaturés linéaires ou ramifiés en C₇-C₃₀, ou aromatiques en C₆-C₃₀, et de monoalcools en C₈-C₃₀, linéaires ou ramifiés ; les mono- et diesters de monoacides carboxyliques linéaires ou ramifiés en C₁₀-C₃₀ et d'éthylèneglycol ; les mono-, di- ou triesters de monoacides carboxyliques linéaires ou ramifiés, saturés ou insaturés, en C₁₀-C₃₀ et de polyol en C₃ ; les mono- ou polyesters de monoacides carboxyliques linéaires ou ramifiés, saturés ou insaturés, ou aromatiques en C₇-C₃₀ et de polyols non polyoxyalkylénés en C₄-C₅₀ ; et les polymères dont au moins l'un des monomères est un ester tels que décrits ci-dessus.

Les esters peuvent être mis en oeuvre en mélange avec un ou plusieurs adjuvants solides ou pâteux, et de préférence pulvérulents. Les adjuvants peuvent être choisis parmi les argiles, les sels, les tensioactifs anioniques, non ioniques, cationiques ou zwittérioniques, les épaississants naturels ou de synthèse, l'amidon éventuellement modifié, les billes de verre, la silice, le nylon, l'alumine, le dioxyde de titane, les zéolithes, le poly(méthacrylate de méthyle) (PMMA), le chitosane, la maltodextrine, la cyclodextrine, les mono- ou disaccharides comme le glucose, le saccharose, le sorbitol ou le fructose, l'oxyde de zinc, de zirconium, les silicabades, le talc, l'acide polyaspartique, les borosilicates notamment de calcium, le polyéthylène, le coton, le polytétrafluoroéthylène (PTFE), la cellulose et ses dérivés, les composés superabsorbants, les carbonates de magnésium ou de calcium, les grains de maïs, le polyacrylamide, l'hydroxyapatite poreux, la soie, le collagène, la sciure de bois, la poudre de fucus, les farines ou extraits de blé, de riz, de pois, de lupin, de soja ou d'orge, la polyvinylpyrrolidone réticulée, l'alginate de calcium, le charbon actif, les particules de poly(chlorure de vinylidène/acrylonitrile), notamment celles commercialisées sous la dénomination générale d'« Expancel® » par la société AKZO NOBEL sous les références particulières « Expancel® WE» ou « DE » Expancels, et leurs mélanges.

Lorsqu'un ou plusieurs adjuvants sont présents, ledit ester ou lesdits esters utilisés dans l'invention sont présents de préférence en une quantité allant de 0,5 à 99% en poids, mieux encore de 1 à 80 % en poids, et encore plus préférentiellement de 2 à 60 % en poids par rapport au poids total ester(s) et adjuvant(s) sous forme solide ou pâteuse.

Lorsque des plantes ou extraits de plantes sont utilisés dans le procédé selon l'invention, ils peuvent être soumis, avant la percolation, à un traitement tel qu'une torréfaction, un cryobroyage, ou une lyophilisation.

La composition de traitement cosmétique des matières kératiniques obtenue selon le procédé de l'invention contient outre le(s) ester(s) et le(s) composant(s) du fluide, à savoir l'eau et/ou le(s) solvant(s) cosmétiquement acceptable(s), éventuellement tout ou partie du ou des adjuvants présents dans le mélange solide ou pâteux.

L'invention concerne également une composition susceptible d'être obtenue par le procédé selon l'invention, la composition particulièrement préférée ne comprenant aucun agent conservateur.

A partir du procédé de préparation de l'invention, on obtient une composition de traitement cosmétique des matières kératiniques qui peut être appliquée directement sur les matières kératiniques, ou qui peut être mélangée à un milieu cosmétiquement acceptable, ou encore au moins un additif classiquement utilisé en cosmétique pourra y être ajouté par un opérateur. On peut également mélanger au moins deux compositions obtenues par le procédé de l'invention. La composition de traitement cosmétique des matières kératiniques résultant éventuellement de mélange(s) et/ou addition(s) indiqués ci-dessus, sera dénommée ci-après composition finale de traitement cosmétique ou composition finale.

Un mode de réalisation particulier de l'invention consiste à appliquer la composition obtenue par l'intermédiaire d'un dispositif ne nécessitant pas d'intervention humaine et équipé éventuellement d'un moyen de refroidissement.

La quantité dudit ester ou desdits esters présents dans la composition finale de traitement cosmétique est en général comprise entre 0,001 à 50% en poids environ, de préférence entre 0,005 et 30% en poids, et encore plus préférentiellement entre 0,01 et 20% en poids du poids total de la composition finale de traitement cosmétique.

Lorsque la composition de traitement cosmétique obtenue par le procédé de la présente invention est mélangée à un milieu cosmétiquement acceptable, un tel milieu est généralement constitué par de l'eau ou par un mélange d'eau et d'au moins un solvant organique pour solubiliser les composés qui ne seraient pas suffisamment solubles dans l'eau.

Par "cosmétiquement acceptable", on entend un milieu compatible avec les matières kératiniques et notamment la peau, les lèvres et/ou les phanères, et qui en outre, présente un aspect, un toucher, une odeur et éventuellement un goût agréable pour l'utilisateur.

A titre de solvant organique, on peut citer, par exemple, les alcools inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ, et encore plus préférentiellement entre 5 et 30 % en poids environ par rapport au poids total de la composition finale de traitement cosmétique.

Au moins un additif classiquement utilisé en cosmétique peut être également ajouté dans les compositions de traitement cosmétique obtenues selon le procédé de la présente invention. A titre d'exemples de tels additifs, on peut citer des agents tensioactifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement différents de ceux décrits ci-dessus tels que, par exemple, des huiles de silicone, des agents filmogènes, des agents conservateurs, des agents opacifiants.

Les additifs ci-dessus sont en général présents en une quantité comprise pour chacun d'eux entre 0,01 et 20 % en poids par rapport au poids de la composition finale.

Bien entendu, l'homme de l'art veillera à choisir ce ou ces éventuels composés complémentaires de manière telle que les propriétés avantageuses attachées intrinsèquement à la composition de traitement cosmétique conforme à l'invention ne soient pas, ou substantiellement pas, altérées par la ou les adjonctions envisagées.

Le pH de la composition finale de traitement cosmétique est généralement compris entre 3 et 12, et de préférence entre 5 et 11. Il peut être ajusté à la valeur désirée au moyen d'agents acidifiants ou alcalinisants habituellement utilisés en cosmétique ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants, on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule suivante : 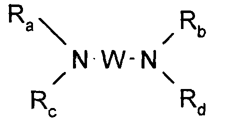 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

La composition finale de traitement cosmétique peut se présenter sous des formes diverses, telles que sous forme de liquides, de crèmes, de gels ou sous toute autre forme appropriée pour réaliser un traitement des matières kératiniques, et notamment de la peau ou des cheveux, et plus particulièrement des cheveux.

La composition finale de traitement cosmétique peut être utilisée, par exemple, comme shampoing, après-shampoing, soin rincé ou non rincé, masque de soin profond, gel douche, lotion ou crème de traitement des matières kératiniques.

La présente invention concerne aussi un procédé de traitement cosmétique des matières kératiniques, comprenant la préparation d'une composition de traitement cosmétique selon le procédé tel que défini ci-dessus, et son application sur les matières kératiniques, par exemple par l'intermédiaire d'un opérateur ou par l'intermédiaire d'un dispositif ne nécessitant pas d'intervention humaine. La durée d'application peut varier entre 15 secondes et 1 heure.

Avant application, la composition de traitement cosmétique obtenue selon le procédé de l'invention, peut être mélangée à un milieu cosmétiquement acceptable et/ou à un ou plusieurs additifs classiquement utilisés en cosmétique tels que décrits ci-dessus.

Un autre mode de réalisation consiste à préparer au moins deux compositions de traitement cosmétique selon le procédé de l'invention, à les mélanger, et à ajouter éventuellement un milieu cosmétiquement acceptable et/ou un ou plusieurs additifs classiquement utilisés en cosmétique tels que décrits ci-dessus, puis à appliquer la composition finale obtenue sur les matières kératiniques.

Les exemples ci-après illustrent la présente invention.

### Exemple 1

On mélange les ingrédients solides suivants dans les proportions indiquées en % en poids par rapport au poids total de mélange solide :
- Myristate de myristyle 30 %
- Méthosulfate de dipalmitoyléthyl-hydroxyéthyl-méthylammonium/alcool cétéarylique vendu sous la dénomination commerciale Dehyquart® F30 par la société COGNIS 30 %
- Palmitostéarate de saccharose 40 %

On place 5 g. de ce mélange dans une machine expresso du commerce. On fait ensuite passer de la vapeur d'eau jusqu'à l'obtention d'une composition (A) présentant un volume final de 50 ml.

On obtient ainsi une composition de traitement cosmétique prête à être appliquée sur les cheveux.

On obtient ainsi un lissage et une douceur améliorées des cheveux.

On peut ajouter deux parties en poids de composition (A), à une partie en poids d'une composition aqueuse (B) contenant 1 % en poids d'hydroxyéthylcellulose pour faciliter l'application.

### Exemple 2

On mélange les ingrédients solides suivants dans les proportions indiquées en % en poids par rapport au poids total de mélange solide :
- Distéarate d'éthylèneglycol 10 %
- Carboxyméthylamidon (fécule de pomme de terre) sous forme de sel de sodium faiblement réticulé, vendu sous la dénomination commerciale PRIMOJEL par la société AVEBE 20 %
- Laurylsulfate de sodium en poudre vendu sous la dénomination commerciale Texapon® Z 95 P par la société COGNIS 70 %

On place 5 g. de ce mélange dans une machine expresso du commerce. On fait ensuite passer de la vapeur d'eau jusqu'à l'obtention d'une composition (A) présentant un volume final de 50 ml.

On obtient ainsi une composition de traitement cosmétique prête à être appliquée sur les cheveux.

On obtient ainsi un lissage et une douceur améliorées des cheveux.

On peut ajouter deux parties en poids de composition (A), à une partie en poids d'une composition aqueuse (B) contenant 1 % en poids d'hydroxyéthylcellulose pour faciliter l'application.

## Revendications

1. Procédé de préparation d'une composition de traitement cosmétique des matières kératiniques, **caractérisé en ce qu'**il comprend une étape de percolation de fluide comprenant au moins de la vapeur d'eau, sous une pression d'au moins 3 bars au travers d'au moins un ester sous forme solide ou pâteuse.

2. Procédé selon la revendication 1, **caractérisé en ce que** le fluide est constitué par de la vapeur d'eau éventuellement accompagnée d'eau liquide, ou encore par un mélange de vapeur d'eau éventuellement accompagnée d'eau liquide, et d'un ou plusieurs solvants liquides et/ou gazeux cosmétiquement acceptables.

3. Procédé selon la revendication 1 ou 2, **caractérisé en ce que** l'ester est choisi parmi les esters de monoacides carboxyliques en C₇-C₃₀, linéaires ou ramifiés, et de monoalcools en C₈-C₃₀, linéaires ou ramifiés ; les esters de monoacides carboxyliques insaturés linéaires ou ramifiés en C₇-C₃₀, ou aromatiques en C₆-C₃₀, et de monoalcools en C₈-C₃₀, linéaires ou ramifiés ; les mono- et diesters de monoacides carboxyliques linéaires ou ramifiés en C₁₀-C₃₀ et d'éthylèneglycol ; les mono-, di- ou triesters de monoacides carboxyliques linéaires ou ramifiés, saturés ou insaturés, en C₁₀-C₃₀ et de polyol en C₃ ; les mono- ou polyesters de monoacides carboxyliques linéaires ou ramifiés, saturés ou insaturés, ou aromatiques en C₇-C₃₀ et de polyols non polyoxyalkylénés en C₄-C₅₀ ; les mono-, di- ou triesters de di- ou triacides carboxyliques linéaires ou ramifiés, saturés ou insaturés, aromatiques ou non, en C₄-C₃₀, hydroxylés ou non, et de mono- ou polyols linéaires ou ramifiés en C₇-C₅₀ ; les mono- ou polyesters d'acides et/ou d'alcools polyoxyalkylénés ou polycérolés, la portion non-oxyalkylénée ou non-glycérolée des acides étant en C₁₀-C₃₀ ; les huiles hydrogénées ou cires hydrogénées ; les sels des acides résultant de l'estérification de monoacides carboxyliques linéaires ou ramifiés, saturés ou insaturés, en C₇-C₃₀ et d'hydroyacides ; les polymères dont au moins l'un des monomères est un ester ; et les polymères résultant de la polycondensation d'au moins un polyacide et d'au moins un polyol.

4. Procédé selon la revendication 3, **caractérisé en ce que** l'ester est choisi parmi le palmitate de cétyle, le myristate de myristyle, l'heptanoate de stéaryle, le béhénate d'isostéaryle, l'iso-octanoate de cétyle, le 2-décylmyristate de 2-décylmyristyle et le bénénate de bénényle ; le benzoate de stéaryle, le ricinoléate de cétyle, l'érucate de 2-octyldodécyle et le benzoate de bénényle ; le monostéarate d'éthylèneglycol, le distéarate d'éthylèneglycol et le dimyristate d'éthylèneglycol ; les mono-, di- et tristéarates de glycéryle, les mono-, di- et tribéhénates de glycéryle, le monomyristate de propylèneglycol, le palmitostéarate de glycéryle, le mono-oléate de glycéryle, le tristéarobéhénate de glycéryle, le tri-undécanoate de glycéryle, le beurre de karité, la cire du Japon, le beurre d'Illipe et le beurre de cacao ; le benzoate de saccharose, le tétra-isostéarate de pentaérythrityle, le tétrabéhénate de pentaérythrityle, le tétralaurate de pentaérythrityle, le palmitate de sorbitane, l'o-octanoyl-6'-D-maltose, le monostéarate de sorbitane, le laurate de saccharose, le dibenzoate de néopentylglycol, le tétrabenzoate de pentaérythrityle, les esters d'acides gras de coco et de sorbitol, le tétrastéarate de ditriméthylolpropane, l'hexastéarate de dipentaérythrityle, les mono et distéarates de méthylglycose, le dilaurate de saccharose, le palmitate de dextrine, les mono- et dipalmitostéarates de saccharose, le sesquistéarate de méthylglucose et le monococoate d'hexylglucoside ; le fumarate de dibéhényle, l'ester citrique de monoglycérides de suif, le citrate de dicocoyl-distéaryl-penarylthrityle, le tartrate de dimyristile, les mucates de dihexyle, dioctyle, dilauryle, dicétyle, les citrates de di- et trilauryle ; l'huile de jojoba à 80 moles d'oxyde d'éthylène (ou OE) et la cire de jojoba à 120 moles d'OE, le myristate de myristyle à 3 moles d'OE, les mono- et distéarates de triéthylèneglycol, les monostéarates de polyéthylèneglycol à 8, 25, 30, 100 moles d'OE, les distéarates de polyéthylèneglycol à 8, 150 et 250 moles d'OE, le dibéhénate de polyéthylèneglycol à 150 moles d'OE, le diisostéarate de polyéthylèneglycol à 90 moles d'OE, les cires d'abeille oxyéthylénées à 6, 8 ou 12 moles d'OE, le monostéarate de sorbitane oxyalkyléné à 4 moles d'OE, le triisostéarate de sorbitane oxyéthyléné à 200 moles d'OE, l'hexastéarate de sorbitane oxyéthyléné à 6 moles d'OE, le dioléate de méthylglucoside oxyéthyléné à 120 moles d'OE, le beurre de karité oxyéthyléné à 75 moles d'OE, l'huile de ricin oxyéthylénée à 25 moles d'OE, le palmitostéarate polyglycérolé à 6 moles de glycérol ; les huiles hydrogénées de colza, de macadamia, de ricin, de coton, de soja, de jojoba, de palme, de son, de riz, de tournesol, la cire de tournesol hydrogénée ; le lauroyl-lactylate de sodium, le béhénoyllactylate de sodium ; les poly(méthacrylates de méthyle), les poly(acétates de vinyle), les polycaprolactones, les poly(acrylates de stéaryle), les poly(acrylates d'éthyle), les copolymères d'acétate de vinyle et d'éthylène, les copolymères acétate de vinyle/versatate de vinyle/acrylate de butyle, les copolymères acide acrylique/méthacrylate de stéaryle, les copolymères N-octylacrylamide/méthacrylate de méthyle/méthacrylate d'hydroxypropyle/acide acrylique/méthacrylate de tertiobutylaminoéthyle, les copolymères acide lactique/vinylpyrrolidone/méthacrylate de lauryle ; et le poly(téréphtalate de néopentylglycol), les polymères de diglycol/cyclohexanediméthanol/isophtalates/sulfoisophtalates, les polyesters de polyglycérol et d'acide eicosanedioique, les polyesters de triméthylolpropane et d'acide diméthylolpropionique, les polymères de polyéthylèneglycol et d'acide adipique, les polymères d'acide sébacique et de butylèneglycol.

5. Procédé selon l'une quelconque des revendications précédentes, **caractérisé en ce que** l'ester sous forme solide ou pâteuse est mis en oeuvre en mélange avec au moins un adjuvant.

6. Procédé selon la revendication 5, **caractérisé en ce que** l'adjuvant est choisi parmi les argiles, les sels, les tensioactifs anioniques, non ioniques, cationiques ou zwittérioniques, les épaississants naturels ou de synthèse, l'amidon éventuellement modifié, les billes de verre, la silice, le nylon, l'alumine, le dioxyde de titane, les zéolithes, le poly(méthacrylate de méthyle) (PMMA), le chitosane, la maltodextrine, la cyclodextrine, les mono- ou disaccharides, l'oxyde de zinc, de zirconium, les silicabades, le talc, l'acide polyaspartique, les borosilicates notamment de calcium, le polyéthylène, le coton, le polytétrafluoroéthylène (PTFE), la cellulose et ses dérivés, les composés superabsorbants, les carbonates de magnésium ou de calcium, les grains de maïs, le polyacrylamide, l'hydroxyapatite poreux, la soie, le collagène, la sciure de bois, la poudre de fucus, les farines ou extraits de blé, de riz, de pois, de lupin, de soja ou d'orge, la polyvinylpyrrolidone réticulée, l'alginate de calcium, le charbon actif, et les particules de poly(chlorure de vinylidène/acrylonitrile).

7. Procédé selon la revendication 5 ou 6, **caractérisé en ce que** le ou les esters sont présents en une quantité de 0,5 à 99% en poids, de préférence de 1 à 80 % en poids par rapport au poids total ester(s) et adjuvant(s) sous forme solide ou pâteuse.

8. Procédé selon l'une quelconque des revendications 5 à 7, **caractérisé en ce que** la composition de traitement cosmétique obtenue contient outre le ou les esters et le(s) composant(s) du fluide, éventuellement tout ou partie du ou des adjuvants présents dans le mélange sous forme solide ou pâteuse.

9. Procédé selon l'une quelconque des revendications précédentes, **caractérisé en ce que** l'étape de percolation est mise en oeuvre avec un fluide sous pression allant de 3 à 30 bars.

10. Procédé selon la revendication 9, **caractérisé en ce que** l'étape de percolation est mise en oeuvre avec un fluide sous pression d'au moins 10 bars.

11. Composition de traitement cosmétique des matières kératiniques susceptible d'être obtenue par le procédé selon l'une quelconque des revendications précédentes.

12. Composition de traitement cosmétique des matières kératiniques selon la revendication 11, ne comprenant pas de conservateur.

13. Procédé de traitement cosmétique des matières kératiniques, **caractérisé en ce que** l'on prépare une composition de traitement cosmétique par le procédé selon l'une quelconque des revendications 1 à 10, et que l'on applique cette composition sur les matières kératiniques.

14. Procédé de traitement cosmétique des matières kératiniques selon la revendication 13, **caractérisé en ce que** l'on applique cette composition sur les matières kératiniques par l'intermédiaire d'un dispositif ne nécessitant pas d'intervention humaine.

15. Procédé de traitement cosmétique des matières kératiniques selon la revendication 13, **caractérisé en ce que**, avant l'application, la composition de traitement cosmétique préparée selon le procédé selon l'une quelconque des revendications 1 à 10, est mélangée à un milieu cosmétiquement acceptable et/ou à un ou plusieurs additifs utilisés en cosmétique.

16. Procédé de traitement cosmétique des matières kératiniques, **caractérisé en ce que** l'on prépare au moins deux compositions de traitement cosmétique selon le procédé défini selon l'une quelconque des revendications 1 à 10, on les mélange et on applique le mélange sur les matières kératiniques.

17. Dispositif de conditionnement d'une composition cosmétique, comprenant un logement fermé délimité par au moins une paroi au moins en partie perméable au fluide sous pression d'au moins 3 bars, ladite composition contenant au moins un ester sous forme solide ou pâteuse.

18. Dispositif selon la revendication 17, dans lequel le logement est délimité par deux feuilles scellées.

19. Dispositif selon la revendication 17, dans lequel le logement est délimité par une barquette fermée par un couvercle.

20. Utilisation de la composition obtenue par le procédé selon l'une quelconque des revendications 1 à 10, pour le traitement cosmétique des matières kératiniques.

21. Utilisation selon la revendication 20, pour le conditionnement des cheveux ou de la peau.
